# EUROPEAN PATENT APPLICATION

(11) **EP 1 025 797 A1**
(43) Date of publication of application: **09.08.2000**
(21) Application number: 99307080.4
(22) Date of filing: 07.09.1999
(51) Int. Cl.: A61B 5/00

(54) **System for locating inflamed plaque in a vessel**

(30) Priority: 08.02.1999 US 246603
(71) Applicant: Brown, David Lloyd, Riverdale, New York 10463 (US)
(72) Inventor: Brown, David Lloyd, Riverdale, New York 10463 (US)
(74) Representative: Thomas, Philip John Duval

(57) **Abstract**

A system for locating inflamed plaque in a vessel is provided herein. The system includes a receiver (18), a positioner (26) and a sensor (22). The receiver (18) receives information about a vessel wall of the vessel. The positioner (26) is used to position the receiver (18) in the vessel to receive information from the vessel wall. The sensor (22) is connected to the receiver (18). In use, the positioner (26) selectively moves the receiver (18) in the vessel to collect information about the vessel wall. The information is then transferred to the sensor (22) to determine the temperature at the vessel wall. Temperatures at various locations can be taken, with elevated temperatures being indicative of inflamed plaque.

## Description

This application is a continuation-in part of applicants' copending application Serial No. 08/774,022, filed on December 27, 1996, entitled "Device and Method for Locating Inflamed Plaque In An Artery", the contents of which are incorporated herein by reference.

### FIELD OF THE INVENTION

The present invention pertains generally to medical devices and methods for evaluating and/or locating plaque in a vessel. The present invention is particularly useful for evaluating and/or locating inflamed or unstable plaque in a vessel.

### BACKGROUND

Plaque can develop in different locations in a patient's cardiovascular system. The plaque can vary in size and shape. For example, the plaque can be quite extensive and occlude a substantial length of the vessel. Alternately, the plaque can be quite short and small.

Further, the condition of the plaque can vary. For example, the plaque can be inflamed and unstable, or the plaque can be quite stable. It is important to recognize that, inflamed and unstable plaque is subject to rupture, erosion or ulceration which can cause the patient to experience a myocardial infarction.

Presently, a number of procedures are available for locating plaque in a vessel. One commonly performed procedure is angiography, which involves taking x-ray pictures of vessels after injecting a radiopaque substance into the vessels. While this procedure is quite effective for locating large plaque in vessels, this procedure is unable to evaluate whether the plaque is inflamed and unstable. Therefore, there is a need of a device and procedure for precisely and accurately locating the position of unstable, inflamed plaque.

In light of the above, it is an object of the present invention to provide a device and method for locating unstable, inflamed plaque in a vessel. Another object of the present invention is to provide a system for determining the size of the unstable, inflamed plaque in the vessel. Another object of the present invention is to provide a device and method for locating inflamed plaque which is relatively easy and inexpensive to manufacture and relatively easy to operate.

### SUMMARY

The present invention is directed to a device and method which satisfies the objectives for locating inflamed plaque on the wall of a vessel of a patient. A device having features of the present invention, includes at least one receiver for receiving information about the patient, a sensor for determining the presence of inflamed plaque based upon the information received from the receiver, and a positioner for selectively positioning each of the receivers in the vessel.

Importantly, unstable and inflamed plaque can cause the temperature of the vessel wall to elevate up to two and a half degrees Centigrade or Celsius proximate the inflamed plaque. With the present invention, each receiver is inserted into the vessel to receive information from the vessel wall. The information is subsequently transferred to the sensor. The sensor determines temperature at each receiver based upon the information received from each receiver. Therefore, the present invention is able to locate inflamed plaque by monitoring the vessel wall for elevated temperatures.

Typically, each receiver includes a single carrier for transferring the information to the sensor. As provided herein, each carrier can be an optical fiber and each receiver can be an aperture formed on the optical fiber which contacts the vessel wall. Preferably, a plurality of receivers are positioned circumferentially around the positioner to decrease the chance of the receivers missing small inflamed plaque.

Optimally, the device includes a radiopaque marker which is positioned proximate each receiver so that the location of the receiver in the vessel can be determined with a fluoroscope.

The sensor can include a monitor, a comparator and an indicator. The monitor displays and/or records temperature at each receiver as the receivers are moved through the vessel. The comparator determines whether a temperature difference exists between each receiver and/or whether a temperature change occurs at each receiver. The indicator indicates when the temperature difference or the temperature change exceeds a predetermined value. Because inflamed plaque can cause the temperature of the vessel wall to elevate up to two and a half degrees Centigrade or Celsius, the predetermined value is typically between 0.5-2.5 degrees Centigrade or Celsius. When the predetermined value is exceeded, the inflamed plaque is located.

The positioner can be an expander which is moveable between a first configuration and a second configuration. Typically, the first configuration is dimensioned for insertion of the receivers into the vessel and the second configuration is dimensioned for positioning the receivers proximate to the vessel wall. An inflatable balloon makes an excellent expander. Additionally, the expander can be used to simultaneously dilate the vessel.

Alternately, for example, the positioner can be a flexible guidewire having a movable section which is adapted to be maneuvered in the vessel. In this embodiment, the receivers are attached to the movable section and the movable section is maneuvered to position the receivers near the vessel wall in the vessel.

The receiver can be implemented in a number of alternate ways. For example, in one embodiment, each receiver could receive infrared radiation from the vessel wall. Alternately, the receiver could include a luminescent material which is positioned near the vessel wall. In this embodiment, the sensor utilizes the change in emissions from the luminescent material to determine temperature. Still alternately, the receiver can utilize ultrasound wave to determine a temperature profile in the vessel.

The invention is also a method for locating inflamed plaque in a vessel of a patient. The method comprises the steps of providing a receiver, positioning the receiver in the vessel of the patient, and determining the existence of inflamed plaque from the information received form the receiver.

It is important to recognize that a device, in accordance with the present invention can accurately locate inflamed plaque by locating elevated temperatures of the vessel wall. Thus, the inflamed plaque may be treated prior to the life threatening rupture or ulceration.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a perspective view of a device having features of the present invention;
Figure 2 is a perspective view of a second embodiment of a device having features of the present invention;
Figure 3 is a side cut-away view taken on line 3-3 and positioned in a vessel;
Figure 4A is a side cut-away view taken on line 4-4 of Figure 1 with a positioner in a first configuration;
Figure 4B is a cut-away view taken of line 4-4 of Figure 1 with the positioner in a second configuration;
Figure 5A is a side cut-away view of a third embodiment of a device having features of the present invention with a positioner in a first configuration;
Figure 5B is a side cut-away view of the embodiment of Figure 5A with the positioner in a second confirmation;
Figure 6A is a side cut-away view of another embodiment of a device having features of the present invention positioned in a portion of an vessel;
Figures 6B is an enlarged view of a portion of the device of Figure 6A;
Figure 7A is a perspective view, in a partial cut-away, of another embodiment of the present invention;
Figure 7B is an enlarged cut-away view taken of line 7B-7B in Figure 7A;
Figure 8A is a perspective view of another embodiment of the present invention;
Figure 8B is an enlarged illustration of another embodiment of the present invention;
Figure 8C is an enlarged illustration of yet another embodiment of the present invention; and
Figure 9 illustrates a side cut-away view of a balloon catheter positioned in a vessel.

### DESCRIPTION

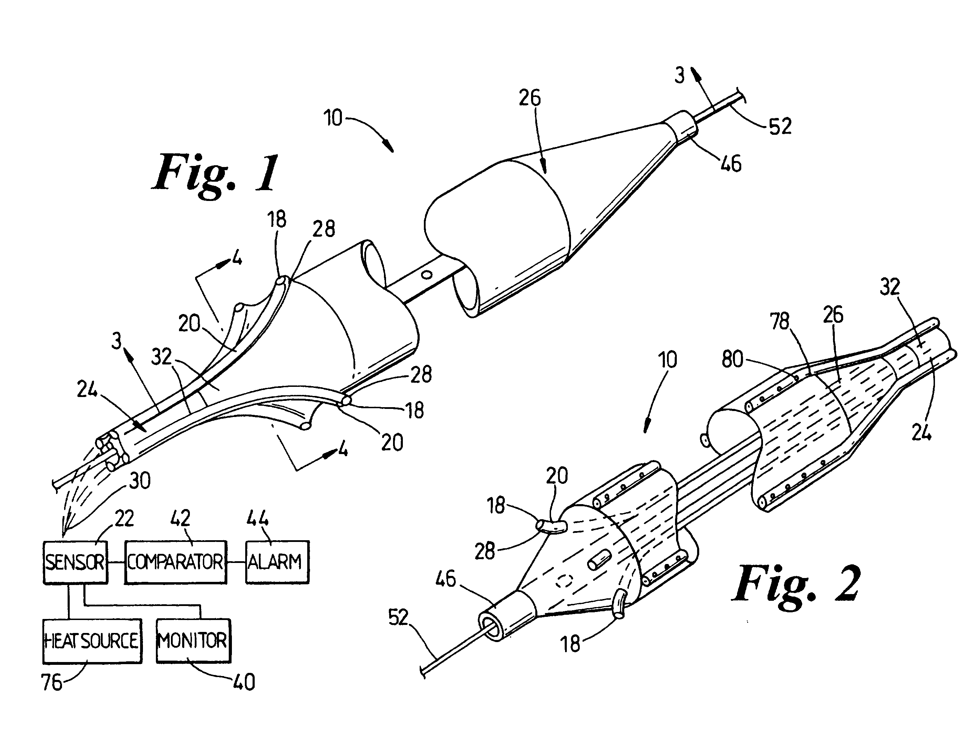

The present invention is a device 10 and method which are particularly suited for locating unstable, inflamed plaque 12 on a vessel wall 14 of a vessel 16. In the embodiment illustrated in Figure 1, the device 10 includes at least one receiver 18, at least one carrier 20, a sensor 22, a catheter 24, and a positioner 26. The temperature of the inflamed plaque 12 is elevated approximately 0.5 to 2.5 degrees Centigrade or Celsius. The present device 10 locates the unstable, inflamed plaque 12 by measuring the temperature of the vessel wall 14 as the device is moved through vessel 16 and locating areas of elevated temperature.

In the embodiment illustrated in the Figures 1-5, each receiver 18 receives information, i.e. infrared radiation, from the vessel wall 14 and each receiver 18 is an aperture proximate a carrier distal end 28 of each carrier 20 which exposes the carrier 20 to the vessel wall 14. Alternately for this embodiment, for example, each receiver 18 can be a projection (not shown) which extends between the vessel wall 14 and the carrier 20.

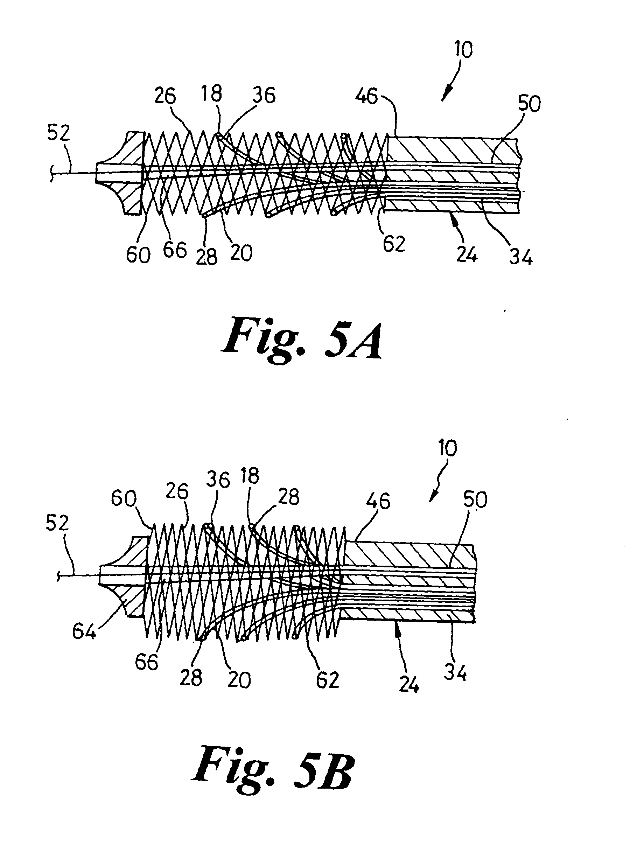

As illustrated in Figure 1, the device 10 can include a plurality of spaced apart receivers 18 positioned circumferentially around the positioner 26 so that the temperature can be monitored around the circumference of the vessel wall 14. For this embodiment, the sensitivity of the device 10 increases as the number of receivers 18 increases, because small inflamed plaque 12 is less likely to pass between the receivers 18. In the embodiments shown in the Figures 1-4, the device includes four, circumferentially, spaced apart receivers 18. Importantly, the positioning of the receivers 18 on the positioner 26 can vary. For example, as shown in Figures 5A and 5B the device 10 can also include a plurality of receivers 18 spaced apart axially along the positioner 26 for additional sensitivity. Also, the receivers 18 can be staggered along the positioner 26.

In the embodiment illustrated in Figures 1-4, each receivers 18 is located above the surface of the positioner 26. Alternately, for example, each receiver 18 may be located on, within or beneath the surface of the positioner 26.

The carrier 20 transfers the information from each receiver 18 to the sensor 22. In the embodiments shown in the Figures 1-5, each receiver 18 includes a separate carrier 20 for transferring infrared radiation to the sensor 22. As provided herein each carrier 20 can be an optical fiber having an aperture at the carrier distal end 28 which forms each receiver 18. As shown in phantom in Figure 1, a carrier proximal end 30 of each carrier 20 is attached to the sensor 20. For this embodiment, any optical fiber which transmits infrared radiation should make a suitable carrier 20.

The carrier 20 can be secured to the catheter 24 and positioner 26 in a number of alternate ways. For example, in the embodiment shown in Figures 1, 3, 4A and 4B, the carriers 20 are positioned and secured to an outer surface 32 of the catheter 20 and the positioner 26. Alternately, in the embodiment shown in Figure 2, the carrier 18 can extend through apertures in the positioner 26. In yet another embodiment shown in Figures 5A and 5B, the carrier 20 can extend through a first lumen 32 of the catheter 24 and through the positioner 26.

Preferably, the device 10 includes a marker 36 positioned proximate each receiver 18 so that the location of each receiver 18 can be determined. For example, each marker 36 can be a radiopaque material, such as silver which is deposited on each carrier 20 proximate each receiver 18. In this version, the position of the radiopaque marker 36 is visible with x-rays and a fluoroscope.

The sensor 22 receives the information from each receiver 18 through each carrier 20 and determines temperature at each receiver 18 based upon the information received or determines if a change of temperature occurs at each receiver 18. Preferably, the sensor 22 receives information from a plurality of receivers 18 and determines the temperature or a temperature difference at each receiver 18. For the embodiment illustrated in Figure 1, the sensor 22 may be any suitable infrared radiation sensor. For example, a sensor 22 made with a suitable pyroelectrical material can be utilized. As is well known to those skilled in the art, pyroelectric material generates an electric charge that is related to the amount of temperature change in the pyroelectric material.

The sensor 22 can include a monitor 40, a comparator 42 and an indicator 44. The monitor 40 displays and/or records the temperature at each receiver 18 for review as the device 10 is moved in the vessel 16. The comparator 42 compares the temperature between the receivers 18 to determine whether a temperature difference exists between the receivers 18. Further, the comparator 42 also compares the temperature at each receiver 18 to determine whether a temperature change occurs at each receiver 18 as the device 10 is moved in the vessel.

The indicator 44 is connected to the comparator 42 and notifies the user of the device 10, i.e., a surgeon, when the temperature difference between each receiver 18 or the temperature change exceeds a predetermined value. For example, if the temperature difference or the temperature change is above the predetermined value, e.g., approximately 0.5-2.5 degrees Centigrade or Celsius, the indicator 44 will notify the user. The indicator 44 can be implemented in a number of alternate ways, such as, an audio signal, i.e., a bell, or a visual signal, i.e., a digital readout.

The catheter 24 can be used to position the positioner 26 and the receivers 18 in the proper location in the vessel 16. Typically, the catheter 24 is cylindrical or elongated shaped and has a catheter distal end 46 which is inserted into the vessel 16 and a catheter proximal end (not shown) which is outside the vessel 16 for manipulating the catheter 24 in the vessel 16. Preferably, the catheter 24 is formed from a flexible and somewhat stiff material such as PET to facilitate movement through the vessel 10.

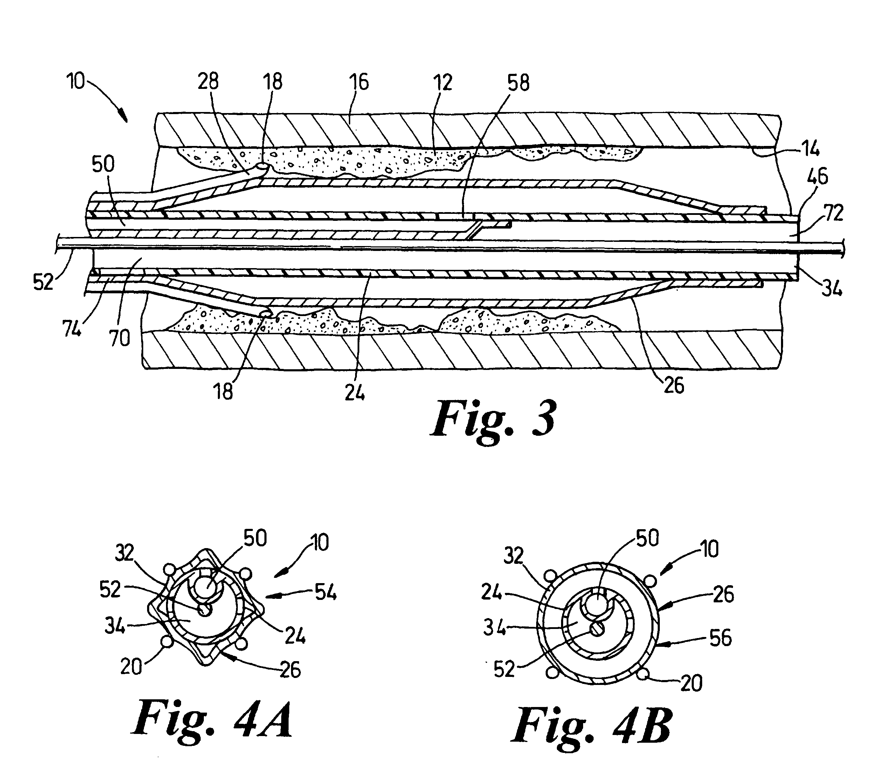

The design of the catheter 24 varies according to the design of the expander 26. For example, the catheter 24 can include the first lumen 34 (as discussed previously) and a second lumen 50. Referring to Figure 3, the first lumen 34 can carry a guidewire 52 for guiding the catheter 24 in the vessel 16 or as shown in Figures 5A and 5B can retain the carriers 20. As discussed below, the second lumen 50 can facilitate movement of the positioner 26 between a first configuration 54 (shown in Figure 4A) and a second configuration 56 (as shown in Figure 4B).

The positioner 26 positions the receivers 18 proximate the vessel wall 14. Further, some of the positioners 26 provided herein can also be used to dilate the vessel 16. In the embodiments illustrated in Figures 1-5, the positioner 26 is an expander which moves between the first configuration 54 for insertion into the vessel 16 and the second configuration 56. As shown in Figures 1-4, the positioner 26 can be an inflatable balloon attached proximate to the catheter distal end 28. Referring now to Figure 3, fluid (not shown) may pass from a pressurized fluid source (not shown) through the second lumen 50 and a balloon aperture 58 in the second lumen 50 to selectively inflate the expander 26. Inflation of this nature may be appreciated by comparison of Figure 4A, where the balloon is shown in the first configuration 54, and Figure 4B, where the balloon is shown substantially in the second configuration 56. For the purposes of the present invention, numerous devices, e.g., pumps or syringes may be adapted to function as a source of fluid pressure.

It may be seen in Figure 3, that when the positioner 26 moves towards its second configuration 56, each receiver 18 contacts the vessel wall 14. It may be appreciated that the positioner 26 may be expanded more or less than the expansion shown in Figure 3.

Alternate embodiments of the positioner 26 are also possible. For example, as shown in Figures 5A and 5B, the positioner 26 can be a cylindrical sleeve that is attached to the catheter distal end 46. The cylindrical sleeve is preferably formed from a wire mesh and has a sleeve distal end 60 and a sleeve proximal end 62. The sleeve proximal end 62 is attached to the catheter distal end 46. A grommet 64 is attached to the sleeve distal end 60. An actuator wire 66 can pass through the second lumen 50 and connect to the grommet 64.

In this embodiment, the guidewire 52 extends through a positioning guidewire lumen in the actuator wire 66. The actuator wire 66 is movable within the second lumen 50 to cause the grommet 64 to move translationally. Translational movement of the grommet 64 moves the sleeve distal end 60 translationally towards, or translationally away from, the catheter distal end 46. Movement of this type may be visualized by comparison of Figure 5A and Figure 5B. In particular, it may be seen in Figure 5A that cylindrical sleeve has a shorter overall length and increased overall width over the cylindrical sleeve illustrated in Figure 5B. In this fashion, the actuator wire 66 may be manipulated to selectively expand the cylindrical sleeve.

The device 10 can also include at least one flow passageway 70 which allows for the flow of fluids, e.g., blood past the expander 26 when the expander 26 is proximate the second configuration 56. Referring to Figure 3, the flow passageway 70 can include a first port 72 and a second port 74 which are in fluid communication with the first lumen 34 and the vessel 16 on each side of the expander 26.

Alternately, in the embodiment shown in Figures 5A and 5B, a series of apertures (not shown) can be formed in the grommet 64 which allows for the passage of fluid, e.g., blood past the expander 26. In yet another embodiment, the expander 26 can be ribbed (not shown) or include grooves (not shown) which form the flow passageway 70 and allow for the flow of blood past the expander 26.

Preferably, the device also includes a heat source 26 which can be connected to the carriers 20 for heating the inflamed plaque 12. In certain situations, it is desirable to treat inflamed plaque 12 with heat. Therefore, the present invention allows the inflamed plaque 12 to be treated almost immediately. The amount of heat which can be applied to the plaque 12 can vary. It is anticipated that a heat source 26 which supplies sufficient heat through the carriers 20 to heat the vessel wall 14 to about 40-45 degrees centigrade is desirable.

Additionally, referring to Figure 2, the positioner 26 can also include one or more fluid passageways 78 having opening 80 for delivering fluid medications to the inflamed plaque 12. This allows positioner 26, for example to immediately apply medications to the inflamed plaque 12 which can seal the inflamed plaque 12, thereby inhibiting erosion or rupture. An inflatable balloon having delivery conduits is disclosed in U.S. Patent No. 5,336,178, Kaplan et al. which is incorporated herein by reference.

Further, it is anticipated that the positioner 26, in some instances, can be expanded to preform angioplasty or deliver a supporting stent (not shown) if necessary.

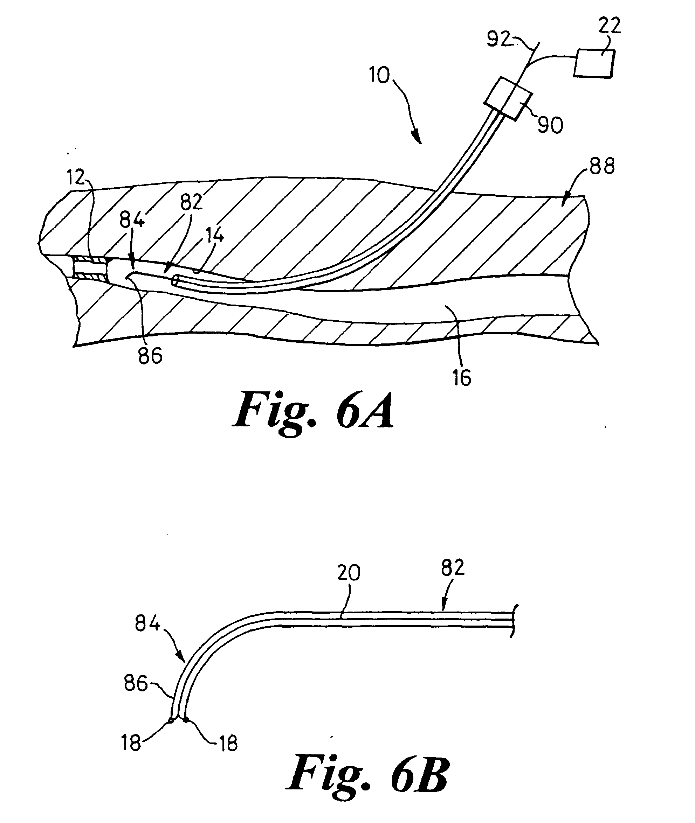

In an alternate embodiment illustrated in Figures 6A-6B, the positioner 26 can be a positioning guidewire 82. In this embodiment, one or more receivers 18 can be attached directly to the positioning guidewire 82. More specifically, the receivers 18 are secured on a movable section 84 near a distal end 86 of the positioning guidewire 82. The movable section 84 can be maneuvered so that the receivers 18 contact the vessel wall 14. Figure 6A illustrates the positioning guidewire 82 operationally positioned within the vessel 16 of a patient 88. In this embodiment, a guiding catheter 90 is used to extend through the patient 88 into the vessel 16. In the embodiment illustrated, the movable section 84 is a bend in the positioning guidewire 82. The movable section 84 is maneuvered through the guiding catheter 90 into the vessel 16. Subsequently, a proximal end 92 of the positioning guidewire 82 is maneuvered and/or torqued until the movable section 84 is near or in contact with the vessel wall 14. In this position, the receivers 18 can detect the temperature at the vessel wall 14. Subsequently, the information can be transferred from the receivers 18 to the sensor 22.

Figure 6B illustrates an enlarged view of the movable section 84. In this embodiment, a pair of receivers 18 are secured to the movable section 84 while a carrier 20 transfers the information from the receivers 18 to the sensor 22. Similar to the embodiments described above, each carrier 20 can be an optical fiber having an aperture which forms the receiver 18. The carrier 20 can transfer infrared radiation to the sensor 22. The carrier 22 illustrated in Figure 6B extends through a center of the positioning guidewire 82. Alternatively, the carrier 22 can run along an outer surface of the positioning guidewire 82 and be attached with an epoxy or shrink wrap (not shown). Further, any number of receivers 18 can be attached to the positioning guidewire 82.

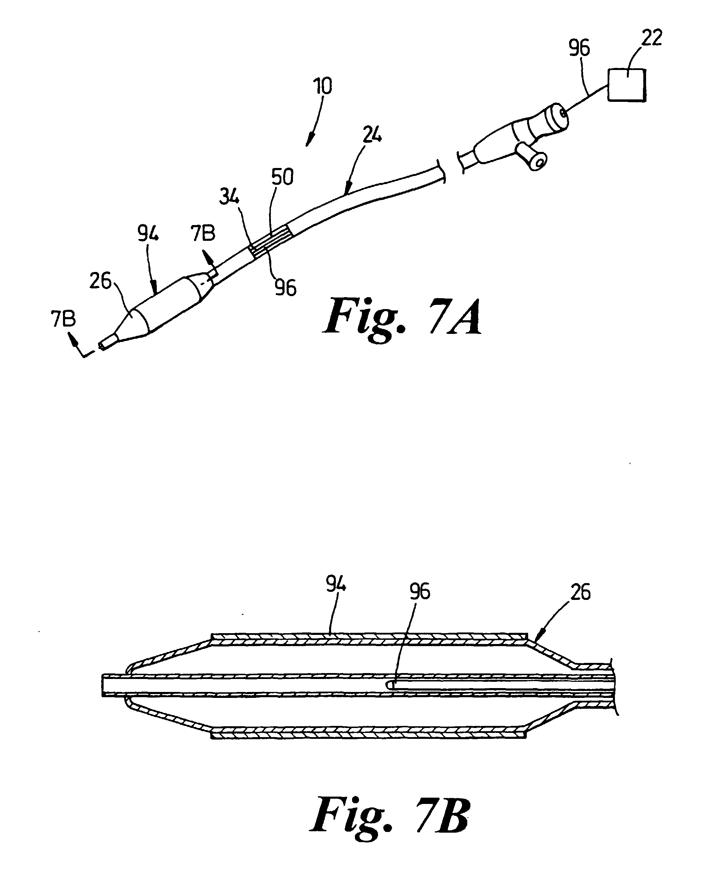

In yet an alternate embodiment illustrated in Figures 7A-7B, the device 10 includes the positioner 26, i.e. an inflatable balloon and a catheter 24 having a first lumen 34 and a second lumen 50. In this embodiment, the receiver 18 includes a coating 94 which coats the positioner 26 and an optical fiber 96 positioned in the first lumen 34. The coating 94 preferably includes a luminescent material such as magnesium germanate or magnesium flourogermanate activated with tetravalent manganese. A more detailed description of the luminescent material can be found in U.S. Patent No. 4,652,143, the contents of which are incorporated herein by reference. The coating 94 can be positioned around a portion or the entire circumference of the balloon. The coating 94 is subsequently positioned near or in contact with the vessel wall 14. In this embodiment, light can be emitted from the optical fiber 96 positioned within the coating 94. The vessel wall 14 excites the luminescent material in the coating 94 and causes the luminescent material to emit radiation with characteristics that are proportional to the temperature of vessel wall 14. Subsequently, the optical fiber 96 receives the information from the luminescent material and transfers the information to the sensor 22. From this information, the sensor 22 is able to determine the presence of inflamed plaque 12. Alternately, for example, separate optical fibers (not shown) can be used to illuminate and receive information in this embodiment.

In still another embodiment illustrated in Figures 8A-8C, the device utilizes sound waves to locate the inflamed plaque. More specifically, the device 10 utilizes ultrasound to plot or make thermal measurements of the vessel wall 14. In particular, the speed of sound is modified by the temperature of the medium through which the sound waves are directed. Thus, the sound waves can be used to determine a temperature profile for the vessel 16 and/or locate areas with higher temperatures. With this information, the inflamed plaque 12 can be located along the vessel 16.

In this embodiment, the device 10 includes the positioner 26 and one or more receivers 18. The positioner 26 is a shaft which carries the receivers 18. In the embodiment illustrated in Figures 8A-8C, each receiver 18 is a transducer which produces sound waves from a voltage signal. Subsequently, the sound waves are received by the transducer to produce an electrical signal. Subsequently, the electrical signal is transferred to the sensor 22 to establish a temperature profile for the vessel 16. A suitable transducer is made of crystal such as P2T (lead zirconate-tifanate). Alternately, for example, separate transducers could be used for producing and receiving the sound waves.

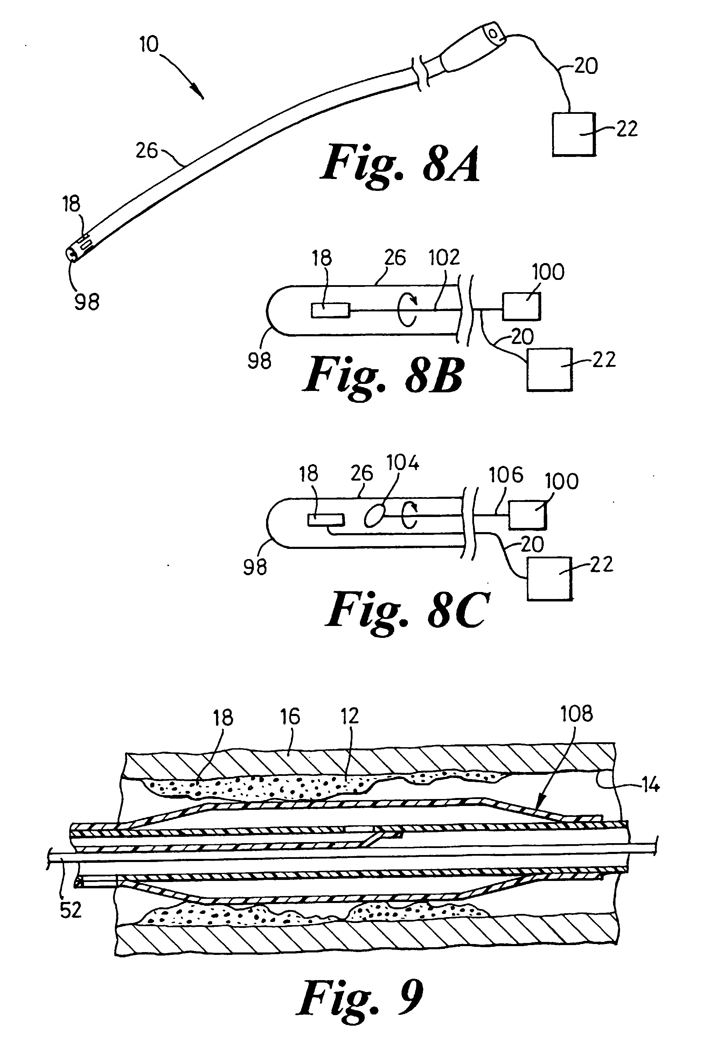

Three versions of this sound wave embodiment are illustrated in Figures 8A-8C. More specifically, in Figure 8A, the device 10 includes a plurality of spaced apart receivers 18 which are positioned near a distal end 98 of the positioner 26. Alternately, the embodiment in Figure 8B, the device 10 includes a single receiver 18 which is rotated by a receiver motor 100 which is coupled to the receiver 18 with a drive shaft 102. In the alternate embodiment illustrated in Figure 8C, the device 10 includes a single receiver 18. However, in this embodiment, a mirror 104 is rotated by a mirror motor 106 which is coupled to the mirror 104 with a mirror drive shaft 106. This allows for the collecting of information around the circumference of the vessel 16. Those skilled in the art will recognize alternate designs for the device 10 which utilizes ultrasound to located inflamed plaque 12. A more complete discussion of ultrasound is provided in the book entitled, Intravascular Ultrasound, R. Erbel, JRTC Roelandt, J Ge, G Gorge, eds. Martin Dunitz, London 1998, the contents of which are incorporated herein by reference.

Importantly, the device 10 is able to evaluate whether plaque is inflamed and unstable. The inflamed and unstable plaque 12 is subject to rupture and/or ulceration which can cause the patient to experience a myocardial infarction. Figure 9 illustrates a balloon catheter 108 positioned in a vessel 16 adjacent to inflamed plaque 12. In this embodiment, after the inflamed plaque 12 is located by device 10 (not shown in Figure 9), the balloon catheter 108 is used to treat the inflamed plaque 12. Thus, the balloon catheter 108 is used to treat the inflamed plaque 12 after it is located with the device 10. During treatment, the balloon catheter 108 dilates the vessel and induces injury and/or ruptures the inflamed plaque 12. This treatment can prevent subsequent rupture of the inflamed plaque 12.

### OPERATION

The operation of one embodiment of the present invention, is best appreciated with reference to Figures 1 and 3, and begins with insertion of the guidewire 52 into the vessel 16. Next, the device 10 is inserted into the vessel 16 over the guidewire 52, with the positioner 26 in substantially its first configuration 54. The advancement of the device 10 will continue until the positioner 26 is at the position where testing of the vessel 16 is to begin.

Next, the positioner 26 is moved from its first configuration 54 toward its second configuration 56. If the positioner 26 is a balloon, fluid is supplied under pressure through the second lumen 50 to inflate the balloon. The expansion of the positioner 26 functions to move the receivers 18 to contact the vessel wall 14.

Once the receivers 18 are against or near the vessel wall 14, the plurality of receivers 18 begin receiving information from the vessel wall 14. The information is transmitted through the carriers 20 to the sensor 22. The sensor 22 receives the information and determines the temperature at each receiver 18. The monitor 40 displays and/or records the temperature at each receiver 18. The comparator 42 compares the temperature at the receivers 18 to determine if a temperature difference exists between the receivers 18. If the temperature difference exceeds the predetermined value, the indicator 44 notifies the user of the device 10 and the inflamed plaque 12 is located.

Next, the positioner 26 is returned to its first configuration 54 for movement to a different site and then returned to proximate its second configuration 56, with the receivers 18 proximate the vessel wall 14. Alternately, depending upon the design of the positioner 26, the positioner 26 may be moved in the vessel 16 with the receivers 18 proximate the vessel wall 14.

As the receivers 18 are moved in the vessel 16, the sensor 22 continues to determine the temperature at each receiver 18 and the comparator 42 continues to determine whether a temperature difference exists between the receivers 18. Further, during this time, the comparator 42 compares the temperatures to determine if a temperature change occurs at any of the receivers 18. Again, if the temperature difference or the temperature change exceeds the predetermined value, the indicator 44 notifies the user of the device 10.

It is important to recognize that the positioner 26 can be moved between its first and second configurations 54, 56 as necessary to facilitate movement of the device 10 through the vessel 16 and to keep the receivers 18 proximate the vessel wall 14.

It is also important to recognize that the size of the inflamed plaque 12 can also be determined from the temperatures as the device 10 is moved through the vessel 16.

Further, it is anticipated that the present device 10 can be used in conjunction with existing procedures such as angiography to precisely locate inflamed plaque 12.

While the particular device 10 as herein shown and disclosed in detail is fully capable of obtaining the objects and providing the advantages herein before stated, it is to be understood that it is merely illustrative of the presently preferred embodiments of the invention. Therefore, no limitations are intended to the details of construction or design herein shown other than as described in the appended claims.

## Claims

1. A device for locating inflamed plaque on a vessel wall of a vessel of a patient, the device comprising:
at least one receiver insertable into the vessel, the receiver being adapted to receive information from the patient;
a positioner for selectively positioning the at least one receiver in the vessel; and
a sensor for receiving the information from the receiver and determining the presence of inflamed plaque based upon the information received from the receiver.

2. The device of claim 1 comprising a plurality of receivers, the receivers being positioned substantially circumferential around the positioner.

3. The device of claim 1 wherein the positioner positions the receiver near the vessel wall of the patient.

4. The device of claim 1 wherein the receiver receives infrared radiation from the vessel wall.

5. The device of claim 1 wherein the positioner includes a positioning guidewire having a movable section which is adapted to be maneuvered in the vessel so that the movable section can be positioned near the vessel wall in the vessel, and the at least one receiver is attached to the guidewire near the movable section.

6. The device of claim 1 wherein the receiver includes a luminescent material which is positioned proximate to the vessel wall by the positioner and the sensor receives information regarding the emissions from the luminescent material.

7. The device of claim 1 wherein the receiver is adapted to receive sound waves from the vessel wall and the sensor utilizes the information regarding the sound waves to determine the presence of inflamed plaque.

8. The device of claim 1 wherein the sensor monitors temperature at the at least one receiver to determine the presence of inflamed plaque.

9. A device for measuring temperature in a vessel wall of a vessel of a patient, the device comprising:
at least one receiver, insertable into the vessel, for receiving information about the vessel wall;
a positioner for selectively positioning the at least one receiver proximate the vessel wall; and
a sensor for receiving the information from the at least one receiver and determining temperature at the at least one receiver based upon the information received.

10. The device of claim 9 comprising a plurality of receivers, the receivers being positioned substantially circumferential around the positioner.

11. The device of claim 9 wherein the positioner positions the receiver near the vessel wall of the patient.

12. The device of claim 9 wherein the positioner includes a positioning guidewire having a movable section which is adapted to be maneuvered in the vessel so that the movable section can be positioned near the vessel wall in the vessel, and the at least one receiver is attached to the guidewire near the movable section.

13. The device of claim 9 wherein the receiver includes a luminescent material which is positioned proximate to the vessel wall by the positioner and the sensor receives information regarding the emissions from the luminescent material.

14. The device of claim 9 wherein the receiver receives sound waves from the vessel wall and the sensor utilizes the information regarding the sound waves to determine the temperature.

15. The device of claim 9 wherein the sensor monitors the temperature at the at least one receiver to determine the presence of inflamed plaque.

16. A method for determining a temperature at a vessel wall of an vessel, the method comprising the steps of:
providing a receiver insertable into the vessel, the receiver being adapted to receive information regarding the vessel wall
advancing a receiver in the vessel;
transferring the information from the receiver to a sensor; and
determining the temperature of the vessel wall at the receiver with the sensor.

17. The method of claim 16 wherein the step of providing a receiver includes providing a plurality of receivers, the receivers being positioned substantially circumferential around a positioner.

18. The method of claim 16 including the step of positioning the receiver near the vessel wall of the patient.

19. The method of claim 16 wherein the step of advancing the receiver includes the step of using a positioning guidewire having a movable section which is adapted to be maneuvered in the vessel so that the movable section can be positioned near the vessel wall in the vessel.

20. The method of claim 16 wherein the step of providing a receiver includes the step of providing a luminescent material adapted for positioning in the vessel.

21. The method of claim 16 wherein the step of providing a receiver includes the step of providing a receiver adapted for receiving sound waves in the vessel.

22. The method of claim 16 including the step of determining the presence of inflamed plaque with the sensor.

23. A method for locating inflamed plaque on a vessel wall of a vessel of a patient, the method comprising the steps of:
providing a receiver, the receiver being adapted to receive information about the patient;
selectively positioning the receiver in the vessel; and
determining the presence of inflamed plaque based upon the information received from the receiver.

24. The method of claim 23 wherein the step of providing a receiver includes providing a plurality of receivers, the receivers being positioned substantially circumferential around a positioner.

25. The method of claim 23 including the step of positioning the receiver near the vessel wall of the patient.

26. The method of claim 23 wherein the step of positioning the receiver includes the step of using a positioning guidewire having a movable section which is adapted to be maneuvered in the vessel so that the movable section can be positioned near the vessel wall in the vessel.

27. The method of claim 23 wherein the step of providing a receiver includes the step of providing a luminescent material adapted for positioning in the vessel.

28. The method of claim 23 wherein the step of providing a receiver includes the step of providing a receiver adapted for receiving sound waves in the vessel.

29. The method of claim 23 including the step of collecting infrared radiation with the receiver.
